# EUROPEAN PATENT SPECIFICATION

(11) **EP 3 130 342 B1**
(45) Date of publication and mention of the grant of the patent: **12.12.2018**
(21) Application number: 15882179.3
(22) Date of filing: 13.10.2015
(51) Int. Cl.: A61L 27/36, A61L 27/58, A61L 27/14, A61K 47/50, A61L 27/54, C12N 15/87, A61K 48/00, A61K 31/7088, A61F 2/28

(54) **METHOD FOR CREATING A PERSONALIZED GENE-ACTIVATED IMPLANT FOR REGENERATING BONE TISSUE**
VERFAHREN ZUR ERZEUGUNG EINES PERSONALISIERTEN, GENAKTIVIERTEN IMPLANTATS ZUR REGENERIERUNG VON KNOCHENGEWEBE
PROCÉDÉ POUR CRÉER UN IMPLANT PERSONNALISÉ À ACTIVATION GÉNIQUE DESTINÉ À LA RÉGÉNÉRATION DU TISSU OSSEUX

(30) Priority: 10.02.2015 RU 2015104291
(43) Date of publication of application: 15.02.2017
(73) Proprietor: "Nextgen" Company Limited, Moscow 119333 (RU)
(72) Inventor: DEEV, Roman Vadimovich, St.Petersburg 192029 (RU); ISAEV, Artur Aleksandrovich, Moscow 119607 (RU); BOZO, Ilya Yadigerovich, Tverskaya obl. 172110 (RU); KOMLEV, Vladimir Sergeevich, Moscow 117513 (RU); DROBYSHEV, Alexey Yurevich, St.Petersburg 127254 (RU)
(74) Representative: Glawe, Delfs, Moll
(86) International application number: PCT/RU2015/000667
(87) International publication number: WO 2016/130041

(56) References cited:
- WO-A1-97/38729
- WO-A1-2015/002707
- US-A1- 2014 011 162
- BOZO I.IA. ET AL.: 'Lokalnaia gennaia terapiia v kostnoi rekonstruktsii.' MATERIALY 1-GO NATSIONALNOGO KONGRESSA PO REGENERATIVNOI MEDITSINE. 04 December 2013, page 30, XP008182774 Retrieved from the Internet: <URL:http://www.mediexpo.ru/fileadmin/user_ upload/content/pdf/thesis/the sis_nkrm13.pdf>
- BRONSHTEIN D.A. ET AL.: 'Klinicheskie parametry osteo- i miagkotkannoi integratsii dentalnykh implantatov pri raznykh sposobakh fiksatsii pokryvnogo proteza.' MATERIALY 1-GO NATSIONALNOGO KONGRESSA PO REGENERATIVNOI MEDITSINE. 04 December 2013, page 37, XP008182775 Retrieved from the Internet: <URL:http://www.mediexpo.ru/fileadmin/user_ upload/content/ pdf/thesis/thesis_nkrm13.pdf>

## Description

### PRIOR ART

The problem of treating patients with bone defects or bone atrophy is highly pressing in the practice of traumatology and orthopedics, maxillofacial and oral surgery [1-3]. A variety of bone substitutes (osteoplastic materials) exist for healing of low-sized bone defects, there are methods to optimize them by mixing with autogenous bone tissue; platelet-rich plasma; plasma rich in growth factors, etc. [1].

However, particularly pressing and socially important is the unsolved problem of the efficient treatment of patients with large bone defects (over 1 cm3) associated with congenital deformities and abnormal development, traumas, inflammatory diseases, oncologic pathology and the first stages of surgical treatment thereof. In these clinical situations where the bone function as a body organ is impaired to a considerable extent or completely lost, it is impossible to reconstruct the bone continuity using commercially available bone substitutes given the pronounced osteogenic insufficiency. Only autogenous bone, either free or vascularized, a "gold standard" of bone grafting, may be efficient in these cases [4, 5].

Bone grafting with autotransplants is associated with causing a further injury, expanding or creating a new surgical approach, considerably increasing the time of surgery, complications rate, and donor site morbidity. In addition, autogenous bone grafting using microvascular technique can be performed only by highly skilled professionals in the setting of specialized medical institutions having the equipment necessary not only to carry out such operations but also to control the compatibility of vascular anastomoses and timely performance of revisualization procedures. At the same time, despite all difficulties, expended resources and funds, there is a high probability of thrombosis and vascular anastomosis failure causing the loss of autografts [6].

Even in case of absolute indications for autobone grafting, graft retention and its complete integration into the recipient site, the long-term outcome of treatment is not always successful. Firstly, this is explained by a high degree of subsequent bioresorption of the grafted material (up to 40% and even more). Secondly, where the bone defect or the area of bone atrophy have irregular shape, it is impossible to model the autograft (a fibula, a rib, a scapula, cranial bones) precisely to the shape of the defect to be substituted, i.e. in order to the diastasis between all the surface of the introduced material and the walls of the bone defect (bone recipient site) did not exceed 1 mm. This causes insufficient consolidation of the graft in the recipient bed area and also occurrence of post-surgical bone deformations, non-unions or excessive bioresorption of the autograft.

Thus, despite being a "gold standard" of the bone substitute materials, bone autografts have a limited use, are not always efficient and associated with a high frequency of complications. One of the key drawbacks of their use and one of the reasons of their insufficient efficiency is that they cannot be modeled precisely to the shape and size of the bone defect. At the same time, close contact of any bone substitute over the entire surface of the bone defect or the area of bone atrophy and complete immobilization are the fundamental principles of bone grafting [1]. When autobone grafting is infeasible or turned out to be inefficient, medical practitioners have to use distraction osteogenesis, prosthetics or to give up form this kind of treatment that considerably lowers the patients' quality of life.

In this connection, it is highly pressing to develop a new more efficient bone substitute able to replace or at least become an alternative in terms of efficiency to the use of bone autografts including vascularized ones. According to the findings of the inventors' research, this problem may be solved by constructing the gene-activated porous biocompatible and biodegradable materials characterized by exactly fitting with the shape and size of bone defects for substituting of which they were made using any available three-dimensional (3D) printing technology as defined in the appended claims.

### STATE OF THE ART

A variety of ordinary bone substitutes is known, such as allogenic and xenogenic bone matrixes from various processing technologies (demineralized, deproteinized, etc.), calcium phosphate ceramics, silicates, organic acids polymers as well as synthetic analogs or natural components of the bone matrix organic and mineral substances. However, they all have only the osteoconductive action because no biologically active components are contained therein. In this connection, they are used only to substitute the low-sized bone defects (low-volume) because of capability only to optimize the reparative osteogenesis being unable to provide its induction [7]. The products of said group in the form of personalized blocks for large bone defects repair are almost not used in clinical practice.

Another category is the activated bone substitutes that may be divided into the groups of materials containing growth factors (proteins), living cells or gene constructions (nucleic acids) .

Bone substitutes with the growth factors have a moderate osteoinductive action allowing toactivate the reparative osteogenesis. However, since the growth factors are short-living and short-distance, and are known to quickly degrade under inflammatory conditions in the operative wound, the overall efficiency of products appeared to be insufficient to substitute the large bone defects. In this regard, there are not numerous of studies used the three-dimensional printing technology for constructing the bone substitutes of a predetermined size and shape with growth factors, and in all cases low-sized (about 1 cm3) products were produced with far from optimal efficacy [8] .

The examples of another, more efficient approach are associated with the development of personalized tissue-engineered bone grafts. Using various technologies for constructing scaffolds with a predetermined size and shape, the researchers have manufactured personalized matrices that were combined then with living cells. By this way the scaffolds of a predetermined size and shape get some osteogenic capacity and theoretically are able to provide pronounced reparative osteogenesis induction. However, the therapeutic potential of the living cells forming part of the personalized bone grafts is limited by the oxygenation demand. In other words, many studies have proved more efficiency of such 3D-printed tissue-engineered bone grafts of small size (up to 1 cm3) compared to ordinary materials. However, in case of large bone defects substitution no effect has been achieved even with ideal modeling of the scaffold shape and size to the defect geometry because the cell inevitably died without the adequate blood supply.

The closest prior art of the claimed invention are the gene-activated bone substitute consisting of a scaffold and nucleic acids, i.e. gene construction encoding the growth factor(s) [9]. Biodegradable 3D printed scaffolds comprising internal pore structures are known from WO 2015/002707 A1, wherein the authors of said document determined the scaffold porosity that facilitates uniform cell seeding and subsequent vascularized bone formation.

A gene-activated bone substitute comprises a "scaffold-nucleic acid" complex whose components were combined by various techniques: chemical binding [7], using adjuvants (for example, gel biopolymer) [11], immediate inclusion of the nucleic acid in the scaffold during its synthesis process, etc.

Such products have the osteoinnductive action, are not limited by the oxygenation demand because no living cells were contained therein. However, many researchers think that the intensity of their osteoinnductive action and, as a result, their efficiency when used to substitute the bone defects is lower than that of the tissue-engineered bone grafts. The reason is a low transfection efficiency of the recipient site cells by the gene constructs and also the use of the "therapeutic strength" just of one or two factors encoded by the gene constructs to induce the reparative process whereas the cells of tissue-engineered materials have a wider mechanism of action.

Due to the inefficiency and drawbacks of the abovementioned technical approaches, a lot of research groups have taken the road of complicating the products by producing the bone substutess comprising complex carriers and combinations of biologically active components: gene constructions and cells, cells and growth factors, growth factors and gene constructions, and even growth factors, gene constructions and cells in a single item. However, production of such materials is excessively costly and their efficiency in substituting the large bone defects remains insufficient.

### BRIEF DESCRIPTION OF THE DRAWINGS

Fig. 1 schematically shows a personalized gene-activated material suitable for substituting a cranial bone defect in the rabbit: A - top view; B - cross-sectional view through the center in the frontal plane.
Fig. 2 shows a constructed personalized gene-activated material suitable for substituting a cranial bone defect in the rabbit.
Fig. 3 shows a personalized gene-activated material substituted a bone defect; 6.5 months after implantation: 1 - implant; 2 - newly formed bone tissue; A - computed tomography; 3D reconstruction; B - histological image (staining: hematoxylin, eosin).
Fig. 4 shows a personalized material not activated by gene constructions substituted a bone defect; 6.5 months after implantation: 1 - implant, 2 - newly formed bone tissue. Histological image (staining: hematoxylin, eosin).
Fig. 5 schematically shows a personalized gene-activated material with locating fixtures, suitable for substituting a large defect of the rabbit's shin bones.
Fig. 6 shows a personalized gene-activated implant fixed in a bone defect with miniplate and miniscrews.
Fig. 7 shows a personalized gene-activated material with locating fixtures substituted a bone defect; 3 months after implantation: 1 - implant, 2 - newly formed bone tissue. Histological image (staining: hematoxylin, eosin).

### DETAILED DESCRIPTION OF THE INVENTION

The invention is set out in the appended claims. The embodiments of the description which do not fall within the scope of said claims are provided for illustrative purposes only and do not form part of the present invention.

In view of other researchers' experience and the prior art, it would be logical to suppose that the materials, both ordinary and activated, comprising either the growth factors or cells or gene constructs cannot be efficient in large bone defects repair due to the above mentioned aspects of the products mechanism of action and unpredictable efficiency in substituting low- and moderate-sized defects.

However, despite the common opinion and the trends (directed to producing more complicated and multicomponent osteoplastic materials) in development, our research group has taken a different way of combining the surgical and biomedical approaches in order to achieve the optimum result.

The gist of the product and the process for its manufacturing proposed in the present invention is the constructing a personalized matrix of biocompatible and bioresorbable materials and combining it with a biologically active component, the gene constructions. A key aspect of the invention distinguishing it from the closest prior art [9] is the use of 3D-printing processes to produce a personalized product exactly fitting with the shape and size to the recipient site, i.e. the area of bone defect or bone atrophy. In other words, the product is produced in the way that after implantation into the recipient area the diastasis between the material and the congruent bone walls did not exceed 1 mm. 3D-printing processes are used to achieve the personalized parameters and precise correspondence of a size and shape. An additional product component may be a fixing structure (reconstruction plates, screws, miniplates, miniscrews, wires, pins, etc.) incorporated into the material at some stage of manufacturing the implant. The presence of this component is mandatory if a bioresorbable material with insufficient mechanical characteristics was chosen as a scaffold for gen constructions that prevented the reliable fixation in the recipient site with any standard techniques (metal constructions, etc.).

The proposed process for constructing of personalized gene-activated materials includes the following stages the order of which may vary:
1. Determining the exact shape and size of a bone defect or bone atrophy area. Standard radiological methods such as computed tomography, X-ray examination, radiography, etc. may be used to this end.
2. Producing a personalized scaffold of the predetermined shape and size from bioresorbable materials using any 3D-printing processes including stereolithography, photopolymerization, etc.
3. Combining the scaffold with the gene constructions at any stage of producing the personalized scaffold or after producing it.

The first stage is directed to planning the morphometric parameters of the product to be manufactured and requires the same research methods as those used usually in planning a surgical intervention. The most preferred option is computed tomography, an essential part of the of skeletal bones pathology diagnosis providing the data for planning of bone reparation procedures. The information obtained in the course of standard computed tomography may be used to model the shape and size of the bone defect and, accordingly, the shape and size of the personalized gene-activated implant using specially configured software (for example, "3D Slicer" available from NHI, USA). Based on the obtained morphometric information, a master file is formed for the 3D-printer or any other apparatus able to produce a three-dimensional implant with predetermined parameters from the required bioresorbable material. Considering the morphological and functional organization and regeneration of the bone tissue, the most preferred materials for constructing the scaffold may comprise calcium phosphates, hydroxyapatite, collagen, bioactive glass ceramics, organic acids polymers and other materials including combinations thereof. The bioresorbable materials chosen to construct the personalized gene-activated implants may have any aggregative state and physical properties only influencing the choice of a particular 3D-printing process.

The 3D-printing of a scaffold may be accomplished by two fundamentally different methods. The first one is direct printing a scaffold from the chosen material. The second one involves the printing of the shaping elements, the guides, (from suitable materials) followed by their use as molds for "casting" (synthesis) a scaffold of predetermined shape and size.

A crucial stage of producing a personalized gene-activated material is to combine the scaffold and gene constructions (for example, plasmid DNA). This part of the process can also be implemented by a number of methods partly definable by the nature of the bioresorbable material chosen to produce the scaffold. If the carrier is produced from a liquid material (gel, sol, solution) or a material temporally being in a liquid phase, the gene construction may be introduced therein before or during 3D-printing. If a solid material (for example, granulated) is used, gene constructions may be added in a liquid solution or as part of any gel material containing them before or during 3D-printing. The simplest and most feasible method in majority of the cases is to combine the produced personalized scaffold with the gene constructions after 3D-printing. To this end, the gene constructions at various concentrations in the form of a solution or as part of a gel may be incubated under different conditions (temperature, exposure time, mechanical impact) with the "printed" scaffold.

If the gene constructions are combined with the scaffold before or during its 3D-printing, it is preferred to carry out the 3D- printing under sterile conditions, i.e. in class A or B clean rooms. However, if the gene constructions are combined with the scaffold after it has been produced, 3D-printing could be performed in the rooms of any class with subsequent sterilization of the resulting personalized scaffold combining with the gene constructions under sterile conditions later on.

It has been surprisingly found out in our researches partly described in the examples that the optimal results could be achieved using personalized gene-activated material produced by 3D-printing process even in case of large bone defects repair. In other words, it is precisely the congruence and tight adherence of the gene-activated material to all surfaces of the recipient site that enables its efficiency. At the same time, the identical personalized scaffold without gene constructions was completely inefficient and non-personalized gene-activated implant made of the same materials and having a standard size and shape was not efficient enough.

The advantageous effect obtained by the present inventors is likely to be explained by the fact that once implanted into the area of a large bone defect, the gene constructions of the standardly-shaped and sized non-personalized gene-activated material did not directly contact the recipient bed cells and fail to reach the target cells. Moreover, diastasis of more than 1 mm between the bone walls and the gene-activated material provides for most released gene constructions to be destroyed and quickly eliminated by the blood clot enzymes and the inflammatory liquid feeling this space. At the same time, the migration of resident cells into the implant is not active enough due to significant space between the product surface and the bone defect walls. As a result, considering the low transfection efficiency, especially in case of a plasmid DNA, nucleic acids do not enter the cells in quantities sufficient to enable a therapeutic effect.

In the contrast, the personalized gene-activated material adheres tightly to all surfaces of the recipient bed with a "free space" being less than 1 mm in length. This facilitates, on the one hand, a faster and more massive migration of cells into the product structure and, on the other hand, shortening of the distance that have to be covered by the gene constructions on the way to the target cells. Due to the lack of space between the gene-activated material and the bone defect walls, this shortening of the distance provides the preservation of more gene constructions, which is extremely important for achieving a therapeutic effect.

Therefore, the full compliance between the product's shape and size and the recipient bed parameters precisely in case of the gene-activated material is of key importance for achieving the product's therapeutic effect. Finding out this fact has become a kind of discovery that allowed us to develop the bone substitute efficient for large (of more than 1 CM3) bone defects repair. However, detailed mechanisms of the discovered efficiency of precisely personalized gene-activated materials need further research and elaboration.

Having solved in part the problem of the large bone defects substitution in the aspect of biomedical methods, we faced another problem, a surgical one. The problem is that the gene-activated material to implement its osteoinductive action has not only to adhere tightly to all recipient bed surfaces immediately after implantation but also to remain in such position all the time until complete integration with the surrounding bone tissue. In other words, the personalized bone graft has to be securely fixed in the recipient site otherwise it will inevitably shift and the spaces will form between the implant and the recipient area that could impair the biological action, give rise to motility and even fall-out. This problem is solved easily in cases where the scaffold consists of a mechanically strong material allowing a standard fixation (screws, miniscrews, microscrews, pins, needles) - any fixtures could be screwed or inserted therein directly during the surgery without destroying the implant. However, the scaffold materials are often fragile and not strong enough as it was in some of the researches we conducted. For example, porous scaffolds of calcium phosphates were broken easily at the attempt to drilling for fixation. It is extremely difficult or just impossible to fix such materials directly during the surgery.

For this point, we developed additional process stages and variants of the personalized gene-activated materials made of the scaffolds with lack of mechanical strength. The solution is to localize the fixation elements into the personalized gene-activated bone graft within the stages of the scaffold manufacturing that can be performed in two ways. The first one consists in introducing a special core made of a metal or a strong bioresorbable material into the interior scaffold structure that may have the holes for fixation elements (screws, miniscrews, microscrews, pins, needles, etc.). The channels leading to the core (or holes in the core) should be formed at the implant surface where the product is supposed to be fixed from. The second option is to place an external fixation system (for example, a miniplate with miniscrews) at a predetermined position and to produce the scaffold of a predetermined shape and size already with fixation elements. As a result, the personalized gene-activated material may comprise either internal (core) or external fixtures.

Importantly, the fixation elements have to be chosen already at the first stage of the personalized gene-activated implant production based on the surgical plan proposed by medical practitioner. To this end, a 3D-bone model may be initially manufactured with the area of defect, atrophy or pathological site whose correction will entail a bone defect formation. This model has to be made available to the medical practitioner planning the surgery. A physician will reproduce the planned manipulations (resection of a bone fragment, grinding of the bone defect walls, etc.) and locate the fixation elements (structures made of metals or strong bioresorbable materials) on the model for immobilizing the bone fragments and the personalized gene-activated implant. The model with the locating fixtures secured thereon at a correct position is used to calculate the morphometric parameters of the personalized gene-activated implant its manufacturing.

### EXAMPLES

### Example 1

### Personalized gene-activated material without "embedded" fixation elements

Before to manufacture one of the variants of the personalized gene-activated material without fixtures by the method proposed herein, an adequate biological model of the bone defect having critical dimensions had to be defined. We were guided by the following criteria in choosing the research model: 1) the bone defect had to be of maximum dimensions; 2) allowed the block to be reliably fixed without using any metal constructions in the implantation area. Considering the above cited criteria, we developed an experimental model of the rabbit's cranial bones defect having a diameter of 20 mm. Cranial osteotomy was performed with a drill to form the bone defect without damaging the dura mater of brain and preserving 1-mm in wide fragments of the internal cortical plate protruding 1 mm towards the center of the defect at 1, 5, 7 and 11 o'clock projections. Preservation of these bone fragments at said positions as support points in combination with the bone defect dimensions have become the distinctive feature of the model. Such model allowed the personalized gene-activated implant to be immobilized within the bone defect without using additional fixing techniques.

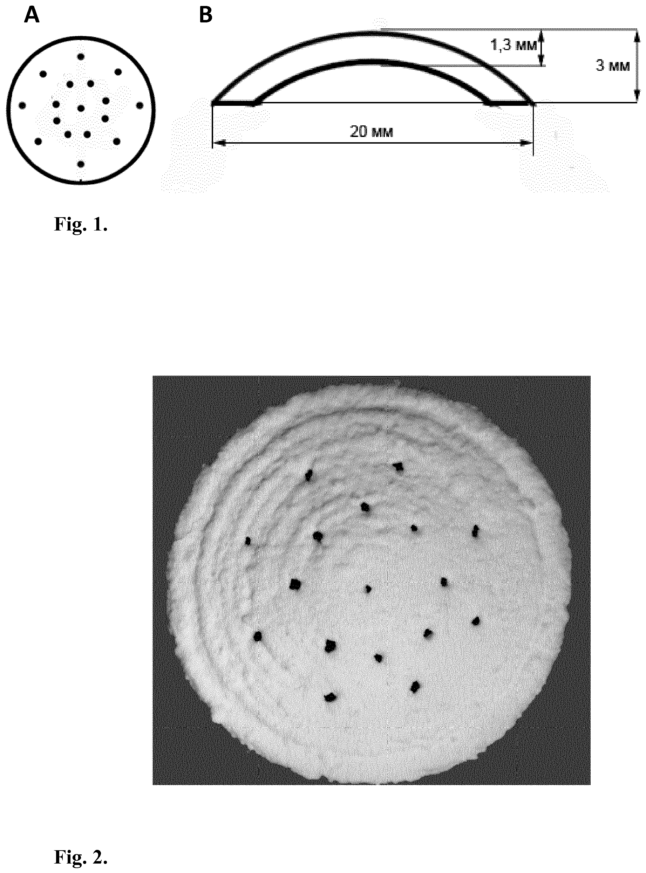

Multispiral computed tomography of the rabbit's cranium was performed before the surgery. Using 3D Slicer software (NHI, USA) we made the manual segmentation of the planned bone defect with the center located at the sagittal suture projection equidistantly from the frontoparietal and parietooccipital sutures. Considering the calculated morphometric parameters of the planned bone defect, 3D-printing of the octacalcium phosphate block was performed. The block was shaped as a convex disk 1.3 mm in thickness, 20 mm in diameter and provided with 17 perforations for cerebral decompression after the cranial bone substitution (Fig. 1, Fig. 2).

The scaffold produced by 3D-printing was combined with a gene construction (a plasmid DNA with a gene encoding the vascular endothelial growth factor (VEGF)) according to the predefined laboratory protocol based on chemically binding the nucleic acids to the scaffold's calcium:
1) washing the scaffold (incubation with 0.5 M phosphate buffer in a 5-ml volume at 37°C with continuing agitation for 12 hours);
2) equilibration (treating with 10 MM phosphate buffer in a 5-ml volume at 37°C with continuing agitation, 3 times for 10 min each time);
3) drying the scaffold (incubation at 37°C until thorough drying for 3 hours);
4) applying the gene constructions (incubation with the plasmid DNA solution in 10 mM phosphate buffer at a concentration of 1 µg/µL at 37°C with continuing agitation for 12 hours);
5) washing the unbound plasmid DNA (treating with 5 MM phosphate solution a 5-ml volume 3 times) off the product;
6) drying (incubation at 37°C until thorough drying for 3 hours).

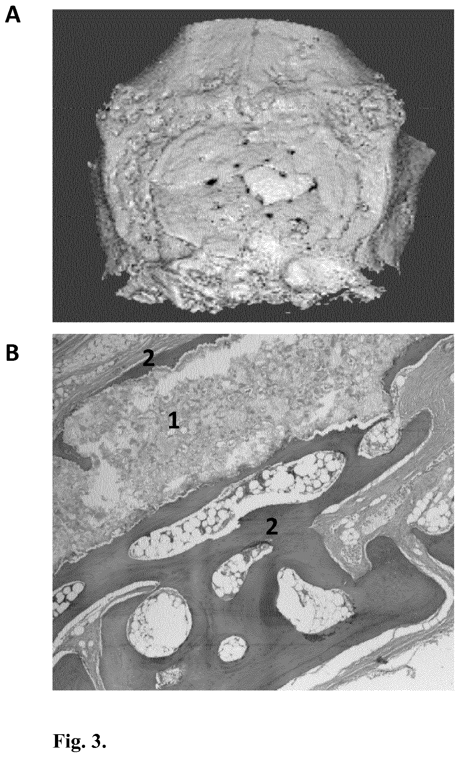

6.5 months after the bone grafting with the personalized gene-activated substitute, the rabbit's cranial bone integrity was completely repaired. The implant did not resorb but its peripheral areas were completely integrated with the surrounding bone tissue. Moreover, a 3-6 mm long newly formed bone tissue was formed along the internal and external surfaces of the personalized gene-activated graft. Both according to the computed tomography and the histological study, the newly formed bone tissue tightly adhered to the implant without forming any connective-tissue interlayer or capsule was detected (Fig. 3).

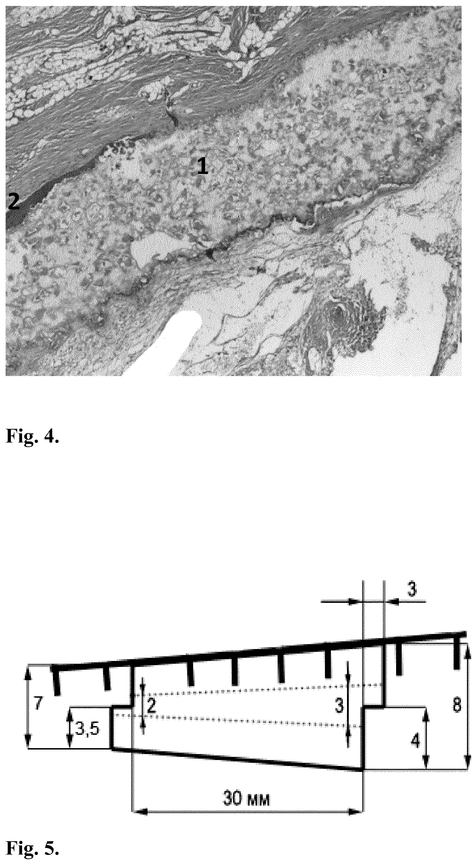

In the absence of gene-activated constructions, the newly formed bone tissue volume was considerably less and the bone proceeding from the periphery did not exceed 1-2 mm (Fig. 4).

### Example 2

### Personalized gene-activated material with "embedded" fixation elements

In order to study this variant of the personalized gene-activated material, we developed another model: a 36-mm long defect of the rabbit's shin bones with stepwise-edged proximal and distal bone fragments.

The personalized gene-activated implant (Fig. 5) was created using the inventive method.

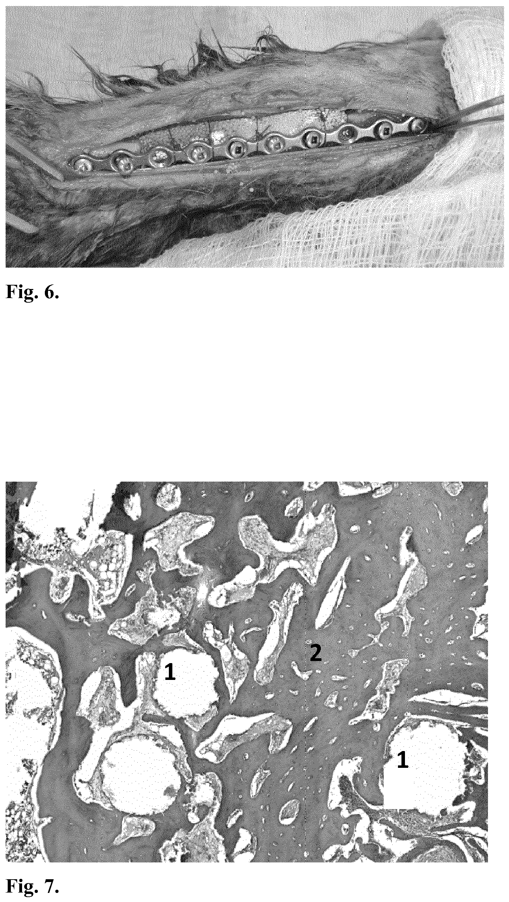

At the first stage, 3D-printing was carried out to manufacture the shaping elements wherein the miniplate and miniscrews intended to fix the implant was positioned. Using the resulting mold, the scaffold was synthesized from tricalcium phosphate exactly fitting with the mold parameters and containing the fixation elements. The implant was combined with the gene construction (a plasmid DNA with vegf and sdf genes (encoding the stromal cell growth factor)) according to the abovementioned protocol. The resulting personalized gene-activated substitute with "embedded" fixation system was implanted into the rabbit's shin bones defect exactly fitting with the implant parameters (Fig. 6).

3 months later, the support ability of the extremity was completely restored. The implant did not resorb by the end of this time but its peripheral areas were completely integrated with the bone fragments (Fig. 7).

Therefore, the method we developed for constructing a personalized gene-activated material and its variants make possible to manufacture the medical product efficient for bone defects substitution including the large ones.

### LIST OF REFERENCES

1. Kulakov L.A., Robustova T.G., Nerobeev L.I., editors. Dental Surgery and Maxillofacial Surgery. National guidance. Moscow, GEOTAR-Media, 2010; 928 p.;
2. Pipitone PS, Rehman S. Management of traumatic bone loss in the lower extremity. Orthop Clin North Am. 2014 Oct; 45 (4) :469-82;
3. Tevlin R, McArdle A, Atashroo D, Walmsley GG, Senarath-Yapa K, Zielins ER, Paik KJ, Longaker MT, Wan DC. Biomaterials for craniofacial bone engineering. J Dent Res. 2014 Dec; 93 (12):1187-95;
4. Al-Nawas B, Schiegnitz E. Augmentation procedures using bone substitute materials or autogenous bone - a systematic review and meta-analysis. Eur J Oral Implantol. 2014 Summer; 7 Suppl 2:S219-34;
5. Nkenke E, Neukam FW. Autogenous bone harvesting and grafting in advanced jaw resorption: morbidity, resorption and implant survival. Eur J Oral Implantol. 2014 Summer; 7 Suppl 2:S203-17;
6. Han Z, Li J, Li H, Su M, Qin L. Single versus dual venous anastomoses of the free fibula osteocutaneous flap in mandibular reconstruction: A retrospective study. Microsurgery. 2013 Sep 3. doi: 10.1002/micr.22176. [Epub ahead of print];
7. Deev R.V., Drobyshev A.Yu., Bozo I.Ya. et al. Construction and biological effect evaluation of gene-activated osteoplastic material with human VEGF gene. Cellular Transplantation and Tissue Engineering, 2013; VIII (3): 78-85;
8. Strobel LA, Rath SN, Maier AK et al. Induction of bone formation in biphasic calcium phosphate scaffolds by bone morphogenetic protein-2 and primary osteoblasts. J Tissue Eng Regen Med. 2014 Mar; 8(3):176-85;
9. Goldstein S.A. In vivo gene transfer methods for wound healing, patent No. 2170104, 10.07.2001;
10. Wegman F., Bijenhof A., Schuijff L. et al. Osteogenic differentiation as a result of BMP-2 plasmid DNA based gene therapy in vitro and in vivo. Eur. Cell Mater. 2011; 21: 230-42.

## Claims

1. A method for constructing a personalized gene-activated implant for bone defects substitution in a mammal, comprising computed tomography of a bone grafting area, modeling a bone defect or bone recipient site based on the computed tomography data, three-dimensional printing of a biocompatible scaffold or a mold for the manufacturing of a biocompatible scaffold, **characterized in that** the biocompatible scaffold
i) is activated by nucleic acids to produce the personalized gene-activated implant and
ii) fits to the shape and size of the bone defect substitution site with a diastasis not exceeding 1 mm between the material of said biocompatible scaffold and the congruent bone walls.

2. The method according to claim 1, **characterized in that** during the manufacturing of the biocompatible scaffold the elements are incorporated therein for subsequent fixation of the product in the implantation area: plates, miniplates, screws, miniscrews, pins, needles, rods and their analogs of metals and bioresorbable materials.

3. A personalized gene-activated implant for substituting the bone defects in a mammal, produced by the method according to claim 1.

4. A personalized gene-activated implant according to claim 3 for use in the treatment of bone defects or bone tissue atrophy in said mammal.

## Patentansprüche

1. Verfahren zur Herstellung eines personalisierten Gen-aktivierten Implantats zur Substitution von Knochendefekten bei einem Säuger, umfassend Computertomographie eines Knochentransplantationsgebietes, Modellierung eines Knochendefekts oder Knochenaufnahmeorts auf der Grundlage der Computertomographiedaten, dreidimensionales Drucken eines biokompatiblen Gerüstes oder einer Form zur Herstellung eines biokompatiblen Gerüstes, **dadurch gekennzeichnet, dass** das biokompatible Gerüst
i) durch Nukleinsäuren aktiviert wird, um das personalisierte Gen-aktivierte Implantate herzustellen und
ii) angepasst ist an die Form und Größe der Knochendefekt-Substitutionsstelle mit einer Diastase von nicht mehr als 1 mm zwischen dem Material des besagten biokompatiblen Gerüsts und den kongruenten Knochenwänden.

2. Verfahren nach Anspruch 1, **dadurch gekennzeichnet, dass** bei der Herstellung des biokompatiblen Gerüsts die Elemente für die anschließende Fixierung des Produktes in Implantationsbereich darin eingebaut werden: Platten, Miniplatten, Schrauben, Minischrauben, Stifte, Nadeln, Stäbe und deren Analoga aus Metallen und bioresorbierbaren Materialien.

3. Personalisiertes Gen-aktiviertes Implantat zur Substitution von Knochendefekten bei einem Säugetier, hergestellt nach dem Verfahren nach Anspruch 1.

4. Personalisiertes, Gen-aktiviertes Implantat nach Anspruch 3 zur Verwendung bei der Behandlung von Knochendefekten oder Knochengewebeatrophie bei besagtem Säugetier.

## Revendications

1. Procédé de construction d'un implant personnalisé activé par voie génique servant à des fins de substitution de déficits osseux chez un mammifère, comprenant la réalisation d'un examen de tomodensitométrie d'une région de greffe osseuse, la modélisation d'un déficit osseux ou d'un site de réception osseux sur la base des données de l'examen de tomodensitométrie, l'impression tridimensionnelle d'un support biocompatible ou d'un moule pour la fabrication d'un support biocompatible, **caractérisé en ce que** le support biocompatible
i) est activé par des acides nucléiques afin de produire l'implant personnalisé activé par voie génique et
ii) est adapté à la forme et à la taille du site de substitution de déficit osseux avec une diastase n'excédant pas 1 mm entre le matériau dudit support biocompatible et les parois osseuses congruentes.

2. Procédé selon la revendication 1, **caractérisé en ce que**, au cours de la fabrication du support biocompatible, les éléments sont incorporés dans celui-ci à des fins de fixation ultérieure du produit dans la région d'implantation : plaques, mini-plaques, vis, mini-vis, chevilles, aiguilles, broches et analogues composés de métaux et de matériaux biorésorbables.

3. Implant personnalisé activé par voie génique servant à des fins de substitution des déficits osseux chez un mammifère, produit au moyen du procédé selon la revendication 1.

4. Implant personnalisé activé par voie génique selon la revendication 3, destiné à une utilisation dans le traitement de déficits osseux ou d'une atrophie de tissu osseux chez ledit mammifère.
